# EUROPEAN PATENT SPECIFICATION

(11) **EP 3 297 604 B1**
(45) Date of publication and mention of the grant of the patent: **22.07.2020**
(21) Application number: 16725094.3
(22) Date of filing: 20.05.2016
(51) Int. Cl.: A61K 31/07, A61K 9/00, A61K 9/107, A61K 47/44, A61P 3/02

(54) **VITAMIN A FOR PARENTERAL ADMINISTRATION**
VITAMIN A ZUR PARENTERALEN VERABREICHUNG
VITAMINE A POUR ADMINISTRATION PARENTÉRALE

(30) Priority: 22.05.2015 EP 15168950
(43) Date of publication of application: 28.03.2018
(73) Proprietor: Fresenius Kabi Deutschland GmbH, 61352 Bad Homburg (DE)
(72) Inventor: BRITO DE LA FUENTE, Edmundo, 61381 Friedrichsdorf (DE); GALLEGOS-MONTES, Crispulo, 61352 Bad Homburg (DE); QUINCHIA-BUSTAMENTE, Lida A., 61352 Bad Homburg (DE); HEKMATARA, Telli, 60316 Frankfurt (DE); BETZ, Michael, 87700 Memmingen (DE)
(74) Representative: Fresenius Kabi Deutschland GmbH
(86) International application number: PCT/EP2016/061359
(87) International publication number: WO 2016/188876

(56) References cited:
- DE-A1- 19 609 476
- US-A- 4 075 333
- Anonymous: "Product information VITALIPID N INFANT AND ADULT", Internet , 3 March 2010 (2010-03-03), XP002751278, Retrieved from the Internet: URL:http://www.medsafe.govt.nz/profs/datas heet/v/VitalipidNinj.pdf [retrieved on 2015-11-23]
- HELEN MACTIER: "Vitamin A for preterm infants; where are we now?", SEMINARS IN FETAL AND NEONATAL MEDICINE, vol. 18, no. 3, 1 June 2013 (2013-06-01), pages 166-171, XP055230659, GB ISSN: 1744-165X, DOI: 10.1016/j.siny.2013.01.004
- GREENE H L ET AL: "Persistently low blood retinol levels during and after parenteral feeding of very low birth weight infants: Examination of losses into intravenous administration sets and a method of prevention by addition to a lipid emulsion", PEDIATRICS, AMERICAN ACADEMY OF PEDIATRICS, EVANSTON, IL, US, vol. 79, no. 6, 1 January 1987 (1987-01-01), pages 894-900, XP009187307, ISSN: 0031-4005
- Anonymous: "Product information INTRALIPID 10%, 20% and 30%", Internet , 18 May 2010 (2010-05-18), XP002751279, Retrieved from the Internet: URL:http://www.medsafe.govt.nz/profs/datas heet/i/Intralipidinf.pdf [retrieved on 2015-11-24]
- WERKMAN S H ET AL: "Effect of vitamin A supplementation of intravenous lipids on early vitamin A intake and status of premature infants", THE AMERICAN JOURNAL OF CLINICAL NUTRITION, AMERICAN SOCIETY FOR NUTRITION, US, vol. 59, no. 3, 1 March 1994 (1994-03-01), pages 586-592, XP009187342, ISSN: 0002-9165

## Description

### Field of the disclosure

The present disclosure relates to pharmaceutical compositions for parenteral administration comprising vitamin A.

The present disclosure further relates to a method for manufacturing the compositions of the disclosure as well as to the use of the compositions of the disclosure.

### Background

Necessary for orderly growth, differentiation of tissues, reproduction, embryonal development and visual function, vitamin A is one of the most important micronutrients.

In the developing world, vitamin A supplementation programmes significantly reduce infant mortality as well as the incidence of xerophthalmia, respiratory infection, and morbidity from gastrointestinal disease. Supplementing newborn infants with vitamin A within 48 hours of birth reduces infant mortality by almost a quarter, with the greatest benefit to those of low birth weight. In the developed world, most infants and children are vitamin A sufficient. Term infants are well supplied with vitamin A in utero, and both human milk and infant formulae contain adequate amounts of vitamin A for normal growth and health. Unfortunately, vitamin A sufficiency cannot be assumed for preterm infants. Born with inadequate body stores of vitamin A and often unable to tolerate routine oral supplementation, they are prone to diseases of the eye and respiratory and gastrointestinal tract (Mactier and Weaver, Arch Dis Child Fetal Neonatal Ed 2005; 90: F103-F108).

Due to their immature gastrointestinal tract, many low birth weight and almost all extremely low birth weight infants cannot absorb and digest enteral nutrition in the first days after birth.

Thus, parenteral administration is the only suitable route for supplementing these infants with vitamin A.

However, under circumstances not allowing for oral supplementation also term infants, children and adults may require parenteral supplementation of vitamin A.

Presently, the recommended doses for parenterally administered vitamin A vary between 700 and 1700 International Units per kg per day.

In order to warrant flexible, individual and independent vitamin A dosing, vitamin A should advantageously be available as a mono vitamin product rather than being fixed part of a pre-mixed multivitamin preparation.

To our knowledge, there is only one such vitamin A product commercially available for parenteral supplementation of vitamin A: Aquasol A™ (Hospira). The product is an aqueous solution of vitamin A and comprises 15 mg retinol (50000 IU, as retinol palmitate) per ml of the composition. Further ingredients are chlorobutanol (as a preservative) and polysorbate (as a solubilizer; retinol palmitate is practically insoluble in water). Aquasol A™ is approved for intramuscular application.

Intramuscular injection, however, is extremely painful, especially to newborn infants, and may cause infections, tissue irritations, muscle damage, haematomas and nerve lesions.

More severely, polysorbates have been associated with the E-Ferol syndrome (thrombocytopenia, renal dysfunction, hepatomegaly, cholestasis, ascites, hypotension and metabolic acidosis) in low birthweight infants.

US5925684 relates to emulsions for parenteral administration comprising at least one carotenoid in a concentration of 0.1 to 10 wt. %. These emulsions are stabilized by means of Poloxamer 188.

Carotenoids have the tendency to stick to plastic surfaces and are thus not ideal vitamin A derivatives for the preparation of storage stable vitamin A compositions.

Poloxamers are synthetic polyoxyethylen/polyoxypropylene block copolymers that may cause allergy and/or intolerance.

Thus there is a need for compositions overcoming the drawbacks described above, i.e. there is a need for safe, compatible and stable compositions suitable for the parenteral supplementation of vitamin A, specifically for compositions that may readily be administered intravenously and be mixed with customary parenteral nutrition products.

### Summary

It has been found that the above mentioned difficulties and drawbacks may be overcome by providing vitamin A in form of an emulsion for parenteral administration. The emulsions according to the present disclosure are stable, even in the absence of polysorbate and polyoxyethylen/polyoxypropylene block copolymers, safe and compatible. Moreover, they may readily be administered intravenously and mixed with customary parenteral nutrition products.

In particular, the present disclosure relates to emulsions for parenteral administration comprising 1000 to 65000, preferably 1500 to 60000 IU, more preferably 20000 to 60000 IU, most preferably 25000 to 55000 IU vitamin A per ml, being free of polysorbate and poloxamer (polyoxyethylen/polyoxypropylene block copolymers).

Particularly preferred embodiments are set forth in the claims.

### Detailed description

### Vitamin A

The term "vitamin A" refers to a group of unsaturated nutritional organic compounds including retinol, retinal, and retinoic acid.

In foods of animal origin, the major form of vitamin A is an ester of retinol, primarily retinyl palmitate, which is converted to retinol in the small intestine. The retinol form functions as storage form of the vitamin and can be converted into its visually active aldehyde form, retinal.
The amount of vitamin A is specified in international units (IU). 1 IU vitamin A is equal to 0.3 µg retinol and 1.8 µg β-carotene.
Vitamin A amounts may also be specified in retinol equivalents (RE). One RE corresponds to 1 µg retinol and 6 µg β-carotene (dissolved in oil).
Preferably, the compositions according to the present disclosure comprise vitamin A in a form other than that of beta-carotene, more preferably in a form other than carotenoid. Most preferably, the compositions according to the present disclosure comprise vitamin A in form of retinyl palmitate.

Vitamin A is the sole vitamin comprised as an active ingredient, i.e. the compositions according to the present disclosure are mono vitamin preparations.

### Emulsion

The compositions according to the present disclosure are emulsions, preferably oil-in-water emulsions, i.e. the continuous phase is aqueous and comprises oil droplets. The emulsion comprises the continuous aqueous phase and preferably 2 wt. % to 30 wt. % of an oil phase based on the total weight of the emulsion. More preferably, the emulsion comprises 5 wt% to 30 wt.% of an oil phase based on the total weight of the emulsion, even more preferably 5 wt.% to 25 wt.% based on the total weight of the emulsion, most preferably 10 wt.% to 20 wt.% based on the total weight of the emulsion. For example, the emulsion comprises 10 wt.% or 20 wt.% of an oil phase based on the total weight of the emulsion.

The aqueous phase comprises water in purity suitable for parenteral administration, i.e. water for injection.

Oil-in-water emulsions for parenteral administration have to be sterile, pyrogen-free, well tolerated, isotonic or as close as possible to isotonicity, free of particulate impurities and storage stable. Their pH should be as close as possible to the pH of the blood.
Because fat globules larger than 5 µm may induce occlusion of the microvasculature, oil-in-water emulsions for parenteral administration must contain only very few oil droplets larger than 5 µm.

The USP refers to this parameter as "PFAT₅", which should not exceed 0.05 (compare USP 36 NF31 <729>).

Scientifically correct, the PFAT₅ value refers to the volume-weighted, large-diameter fat globule limits of the dispersed phase, expressed as the percentage of fat residing in globules larger than 5 µm (PFAT₅).

### The oil phase

The oil phase may comprise a variety of different lipids, e.g. oils, e.g. soybean oil, olive oil, fish oil, fish oil extract, safflower oil, corn oil, sunflower oil, coconut oil, palm kernel oil, rapeseed oil, medium chain triglycerides (MCT) and mixtures thereof.

Preferably, the oil phase comprises one or more oils selected from the group consisting of soybean oil, olive oil, fish oil, fish oil extract and MCT. More preferably, the oil phase comprises soybean oil, olive oil, fish oil and MCT.

The term "fish oil" refers to "purified fish oil" and to "purified fish oil rich in omega 3 fatty acids", the latter according to the European Pharmacopoeia 6.0 comprising at least 9 wt.% of the omega-3-fatty acid docosahexaenoic acid (DHA) and at least 13 wt.% of the omega-3 fatty acid eisosapentaenoic acid (EPA) expressed as triglycerides.

The term "fish oil extract" refers to mixtures highly concentrated in EPA and DHA obtained e.g. from fish oil e.g. by supercritical fluid extraction and subsequent purification via e.g. chromatographic methods. Alternatively, the oil can be extracted using extraction techniques such as the one described in US6750048. Additional extraction and/or purification techniques are described in WO2001/076715 and WO2001/076385. The sum of EPA and DHA contained in these fish oil extracts is at least 500 milligram per gram of extract.

The fish oil extract comprises EPA and DHA in esterified form, e.g. in form of triglycerides or ethyl esters.

The term "medium chain triglycerides" refers to triglycerides of fatty acid being 6 to 12 carbon atoms in length, including caproic acid, caprylic acid, capric acid and lauric acid.

### The emulsifier

The emulsions according to the present disclosure comprise at least one pharmaceutically acceptable emulsifier. The term "emulsifier" refers to compounds which stabilize the composition by reducing the interfacial tension between the oil phase and the water phase and which typically comprise at least one hydrophobic group and at least one hydrophilic group. These emulsifiers (which may also be referred to as surfactants) are preferably used in amounts effective to provide, optionally together with further surfactants present, a stable and even distribution of the oil phase within the aqueous phase. In particular, the emulsifier is selected from the group of emulsifiers that have been approved for parenteral administration.

According to the present disclosure, the at least one emulsifier is lecithin. Within the meaning of the present disclosure the term "lecithin" refers to naturally occurring or synthetic lecithin that may be suitably refined. Suitable lecithins include, but are not limited to, lecithins derived from egg, corn or soybean or mixtures thereof. Lecithins are typically mixtures of diglycerides of fatty acids linked to the choline ester of phosphoric acid and can contain differing amounts of other compounds depending on the method of isolation. Typically, commercial lecithin is a mixture of acetone-insoluble phosphatides. Preferably, the lecithin is obtained from egg or from seeds including soybean and corn, using methods well known in the art. Lecithin obtained from soybean is referred to herein as soy lecithin. Lecithin obtained from egg is referred to herein as egg lecithin. Preferably, the emulsions comprise lecithin as emulsifier, more preferably the lecithin is selected from the group consisting of egg lecithin, soy lecithin, and mixtures thereof.

These are commercially available, e.g. under the trade names Epikurin™ 170 (soy lecithin), PL 90 or Lipoid E80 (both egg lecithin).

Preferably, the lecithin is used in an amount of 0.5 to 5 wt.%, more preferably 0.5 to 3 wt.%, most preferably 1.0 to 2.5 wt.%.

Alternatively, krill oil may be used as an emulsifier. Krill oil is an extract prepared from a species of antarctic krill, Euphausia superba. It has obtained GRAS (generally recognized as safe) status from the FDA and is commercially available, e.g. from Olympic Seafood (Bioriginal Europe/Asia B.V.) and Aker BioMarine Antarctic AS. The emulsifying properties of krill oil mainly rely on its content in phospholipids (including phosphatidylcholine, phosphatidylethanolamine and phosphatidylinositol).

### The antioxidant

The emulsion may comprise at least one pharmaceutically acceptable antioxidant.

An antioxidant useful in the emulsion of the disclosure may be any pharmaceutically acceptable compound having antioxidant activity, for example, the antioxidant may be selected form the group consisting of sodium metasulfite, sodium bisulfite, sodium sulfite, sodium thiosulfate, thioglycerol, thiosorbitol, thioglycolic acid, cysteine hydrochloride, n-acetly-cysteine, citric acid, alpha-tocopherol, beta-tocopherol, gamma-tocopherol, soluble forms of vitamin E, butylated hydroxyanisole (BHA), butylated hydroxytoluene (BHT), t-butylhydroquinone (TBHQ), monothioglycerol, propyl gallate, histidine, enzymes such as superoxide dismutase, catalase, selenium glutathione peroxidase, phospholipid hydroperoxide and glutathione peroxidase, Coenzyme Q10, tocotrienols, carotenoids, quinones, bioflavonoids, polyphenols, bilirubin, ascorbic acid, isoascorbic acid, uric acid, metal-binding proteins, ascorbic acid palmitate, an antioxidant obtained or obtainable from rosemary, rosemary extract and mixtures thereof.

The at least one antioxidant is in particular selected from the group consisting of alpha-tocopherol, beta-tocopherol, gamma-tocopherol, ascorbic acid, and mixtures of two or more thereof.
If present, the total amount of agents with antioxidant activity is preferably in the range of from 0.01 wt.% to 0.05 wt. %, more preferably from 0.01 wt.% to 0.04 wt.%, more preferably from 0.01 wt.% to 0.03 wt.%, and even more preferably from 0.015 wt.% to 0.025 wt.% based on the total weight of the emulsion.

### The tonicity agent

The emulsion may comprise at least one pharmaceutically acceptable tonicity agent.

Tonicity agents are used to confer tonicity. Suitable tonicity agents may be selected from the group consisting of sodium chloride, mannitol, lactose, dextrose, sorbitol and glycerol.

Preferably, the tonicity agent is glycerol.

Preferably, the total amount of tonicity agents is in the range of 0.1 to 10 wt.%, more preferably from 1 wt.% to 5 wt.%, more preferably from 1 wt.% to 4 wt.%, more preferably 1 wt.% to 3 wt.%, more preferably from 1.5 wt.% to 2.8 wt.%, and even more preferably from 2.0 wt.% to 2.8 wt.% based on the total weight of the emulsion.
In case the tonicity agent is glycerol the most preferred amount is 2.0 wt. % to 2.5 wt.% based on the total weight of the emulsion.

Preferably, the emulsion has an osmolality in the range of 305 to 420 mOsmol/kg, measured with a Vapor Pressure Osmometer, Model 5520 (Vapro TM) according to USP <785>.

### pH adjustment

The pH of the emulsion may be adjusted by adding solutions of conventionally known acids or bases such as HCI and NaOH or through the use of buffers, such as phosphate buffers.

The final pH of the emulsion is preferably in the range of from 6 to 9, more preferably between 7.5 and 9.

Preferably, the pH of the emulsion according to the disclosure is adjusted using a solution of NaOH.

### The co-surfactant

The emulsion according to the disclosure may further comprise a pharmaceutically acceptable co-surfactant.

A co-surfactant is an amphiphilic molecule, i.e. a molecule that contains both hydrophilic and lipophilic groups. Usually, a co-surfactant substantially accumulates with the emulsifier at the interfacial layer. The hydrophile-lipophile balance (HLB) number is used as a measure of the ratio of hydrophilic and lipophilic groups present in a surfactant or co-surfactant, respectively. Usually, a co-surfactant with a very low HLB value (thus with a relatively high affinity to oil) is used together with a surfactant with a high HLB to modify the overall HLB of the system. Unlike the emulsifier, the co-surfactant may not be capable of forming self-associated structures, like micelles, on its own. Several kinds of molecules including nonionic emulsifiers, alcohols, amines and acids, can function as co-surfactants in a given system. The co-surfactant is usually used in a lower amount than that of the emulsifier. Apart from modifying the overall HLB value of the system, the co-surfactant has the effect of further reducing the interfacial tension and increasing the fluidity of the interface. Co-surfactants may also adjust the curvature of the interfacial film by partitioning between the tails of the emulsifier chains, allowing greater penetration of the oil between the emulsifier tails.

Preferably, the co-surfactant is a free unsaturated fatty acid or a salt thereof, preferably an omega-9 fatty acid or a salt thereof, more preferably a monounsaturated omega-9 fatty acid or a salt thereof, more preferably oleic acid or sodium oleate.

The total amount of the co-surfactant is preferably in the range of from 0.01 wt.% to 1 wt.%, more preferably in the range of from 0.02 wt.% to 0.5 wt.%, more preferably in the range of from 0.02 wt.% to 0.20 wt.% based on the total weight of the emulsion.

### The co-solvent

The emulsion according to the disclosure may further comprise a pharmaceutically acceptable co-solvent.

The term co-solvent refers to molecules that may increase the stability of the emulsion. In addition to making the environment more hydrophobic by reducing the dieelectric constant of water, co-solvents increase the amount of molecularly dispersed emulsifier and/or co-surfactant in the aqueous phase. Availability of free surfactant aids in the solubilization of hydrophobic molecules by creating pockets of hydrophobic regions within the aqueous phase.

The co-solvent may be selected from the group consisting of ethanol, propylene glycol and polyethylen glycol.

Preferably, the co-solvent is a polyalkylene glycol or an alkylene glycol, preferably polyethylen glycol or polypropylen glycol, more preferably polyethylene glycol (PEG). The PEG preferably has a mean molecular weight in the range of from 100 to 20000 Da, more preferably in the range of from 200 to 1000 Da, more preferably in the range of from 300 to 600 Da, most preferably around 400 Da.

Preferably, the co-solvent is selected from the group consisting of PEG 200, PEG 300, PEG 400, PEG 600, PEG, 1000, PEG 1450, PEG 4000, PEG 6000, PEG 8000 and PEG 20000. Most preferably, the co-solvent is PEG 400.

Preferably, the total amount of co-solvents ranges from 0.1 wt% to 2.0 wt.%, more preferably from 0.25 wt.% to 1.75 wt.%, more preferably from 0.50 wt.% to 1.50 wt.%, more preferably from 0.70 wt.% to 1.40 wt.%, more preferably from 0.80 wt.% to 1.30 wt.%, and even more preferably from 0.90 wt.% to 1.20 wt.% based on the total weight of the emulsion.

### The droplet size

Since the emulsion of the disclosure is an oil-in-water emulsion, the continous phase is aqueous and comprises oil droplets. These oil droplets are stabilized within the aqueous phase by at least one emulsifier and optionally further additives. The size of the oil droplets depends on the qualitative and quantitative composition of the emulsion and its preparation.

The oil droplets of the emulsion herein preferably have a mean diameter of 150 to 350 nm when measured directly upon sterilization using an LS 13 320 Laser Diffraction Particle Size Analyser (Beckman Coulter) according to USP <729>.

### Preparation of the emulsion

The present disclosure also relates to a method for preparing an emulsion for parenteral administration and to an emulsion obtained or obtainable by said method, wherein the method comprises
a) providing an oil phase comprising vitamin A and one or more lipids and optionally at least one pharmaceutically acceptable antioxidant and/or a pharmaceutically acceptable co-surfactant,
b) providing an aqueous phase comprising water for injection and optionally a pharmaceutically acceptable tonicity agent and/or an agent for pH adjustment and/or a pharmaceutically acceptable co-surfactant and/or a pharmaceutically acceptable co-solvent,
c) forming a pre-emulsion by mixing the oil phase provided in step a) with the aqueous phase provided in step b);
d) forming the emulsion by high-pressure homogenizing the pre-emulsion obtained in step c) and
e) sterilizing the emulsion obtained in step d),
wherein the at least one pharmaceutically acceptable emulsifier is added either in step a or in step b.

It is to be understood that any of the optional further components of the emulsion may be added in any of the steps a), b), c) or d) or in one or more additional steps.

### Step a)

Step a) is preferably carried out by mixing one or more lipids and vitamin A and optionally the at least one antioxidant and/or the co-surfactant. This step is preferably carried out at a temperature of 50 to 65 °C, wherein during this step the temperature may be varied or held essentially constant for a maximum 30 minutes until a homogeneous and clear phase is obtained.

It is to be understood that in step a) further additives may be added.

### Step b)

Step b) is preferably carried providing water for injection and optionally adding the tonicity agent and/or the co-surfactant.

The aqueous phase is then heated to a temperature of 55 to 80 °C, preferably for a time of 1 minute to 1 hour, more preferably from 5 to 30 minutes, more preferably from 5 to 15 minutes.

Preferably, step b) further comprises adjusting the pH to values between 7 and 10, preferably to a pH between 8 and 9, preferably by adding a solution of NaOH.
It is to be understood that in step b) further additives may be added.

In particular, it is to be understood that the at least one pharmaceutically acceptable emulsifier - depending on its nature - may be added either in step a) or in step b).

### Step c)

The method further comprises mixing the oil phase provided in step a) with the aqueous phase provided in step b) thereby forming a pre-emulsion. The mixing may be carried out by any method known to those skilled in the art. Preferably, the mixing is carried out using a high shear mixer.

Preferably the oil phase is added to the aqueous phase or vice-versa at a temperature in the range of from 50 to 80 °C.

Preferably the oil phase is added to the aqueous phase or vice-versa at a pressure such as under nitrogen pressure, in the range of from 0.20 to 0.80 bar, more preferably from 0.2 to 0.4 bar. During this step the pressure may be varied or held essentially constant.

According to a preferred embodiment, the mixture is stirred for a time in the range of from 1 minute to 1 hour, preferably from 10 to 30 minutes. During this step, the temperature may be varied or held essentially constant.

It is to be understood that further components may also be added after the formation of the pre-emulsion. According to a preferred embodiment, the pH of the pre-emulsion is adjusted to a pH in the range of from 8 to 10, in particular by adding sodium hydroxide, if necessary.

### Step d)

The method further comprises the homogenization of the pre-emulsion obtained in step c). This homogenization may be carried out by any suitable method known to those skilled in the art.

Preferably the mixture is homogenized at a temperature in the range of from 40 to 70 °C, preferably from 40 to 60 °C, more preferably from 50 to 60 °C.

Preferably, the pre-emulsion is homogenized at a pressure in the range of from 400 to 600 bar, more preferably from 450 to 550 bar. During this step the pressure may be varied or held essentially constant.

Preferably, the homogenization is carried out using a high pressure homogenizer or a microfluidizer.

### Step e)

The method further comprises the sterilization of the emulsion obtained in step d) to ensure its suitability for parenteral administration.

The sterilization may be carried out by any suitable method known to those skilled in the art. In particular, the sterilization is carried out by autoclaving, preferably at a temperature in the range of from 119 to 122 °C, more preferably at a temperature around 121 °C, preferably for a time in the range of from 1 minute to 30 minutes, preferably of from 10 minutes to 15 minutes.

### Route of administration

The compositions according to the present disclosure are adapted for parenteral administration, i.e. for a route of administration "other than via the gastrointestinal tract". This includes for example the intravenous, intra-arterial, intramuscular, intraperitoneal and subcutaneous administration.
Preferably, the compositions according to the present disclosure are administered intravenously.

The present disclosure includes inter alia the following aspects:
In a first aspect the present disclosure relates to an emulsion for parenteral administration comprising 1000 to 65000, preferably 1500 to 60000, more preferably 20000 to 60000, most preferably 25000 to 55000 IU vitamin A per ml, being free of polysorbates and polyoxyethylen/polyoxypropylene block copolymers.

In a second aspect the present disclosure relates to an emulsion according to aspect 1, comprising vitamin A in a form other than beta-carotene, preferably in a form other than carotenoid.

In a third aspect the present disclosure relates to an emulsion according to aspect 1, comprising retinyl palmitate.

In a fourth aspect the present disclosure relates to an emulsion according to aspect 2, wherein the whole amount of vitamin A is provided in form of retinyl palmitate.

In a fifth aspect the present disclosure relates to an emulsion according any of the preceding aspects, wherein vitamin A is the sole vitamin comprised as an active ingredient.

In a sixth aspect the present disclosure relates to an emulsion according to any of the preceding aspects, wherein the oil phase of the emulsion comprises one or more oils selected from the group consisting of soybean oil, olive oil, fish oil, fish oil extract, safflower oil, corn oil, sunflower oil, coconut oil, palm kernel oil, rapeseed oil and medium chain triglycerides (MCT).

In a seventh aspect the present disclosure relates to an emulsion according to any of the preceding aspects, wherein the oil phase comprises one or more oils selected from the group consisting of fish oil, fish oil extract, MCT, soybean oil and olive oil.
In an eighth aspect the present disclosure relates to an emulsion according to any of the preceding aspects, wherein the oil phase of the emulsion comprises fish oil, fish oil, olive oil, soybean oil and MCT.

In a ninth aspect the present disclosure relates to an emulsion according to any of the preceding aspects, comprising a pharmaceutically acceptable tonicity agent, preferably glycerol.

In a tenth aspect the present disclosure relates to an emulsion according to any of the preceding aspects, comprising at least one co-solvent, preferably polyethylene glycol.
In an eleventh aspect the present disclosure relates to an emulsion according to any of the preceding aspects, comprising an agent for pH adjustment, preferably NaOH.
In a twelfth aspect the present disclosure relates to an emulsion according to any of the preceding aspects, comprising at least one pharmaceutically acceptable emulsifier, preferably lecithin.

In a thirteenth aspect the present disclosure relates to an emulsion according to any of the preceding aspects, comprising a pharmaceutically acceptable co-surfactant, preferably an omega-9 fatty acid or a pharmaceutically acceptable salt thereof, more preferably oleic acid or sodium oleate.

In a fourteenth aspect the present disclosure relates to an emulsion according to any of the preceding aspects, comprising at least one pharmaceutically acceptable antioxidant, preferably selected from the group consisting of alpha-tocopherol, beta-tocopherol, gamma-tocopherol, ascorbic acid, ascorbic acid palmitate, an antioxidant obtained or obtainable from rosemary, rosemary extract and mixtures thereof.

In a fifteenth aspect the present disclosure relates to an emulsion according to any of the preceding aspects, wherein the emulsion is an oil-in-water emulsion and wherein the mean diameter of the oil droplets is between 100 and 350 nm.

In a sixteenth aspect the present disclosure relates to an emulsion according to any of the preceding aspects for use in the treatment or prevention of a vitamin A deficiency in adults, children, infants, preterm infants, low birth weight infants, very low birth weight infants or extremely low birth weight infants.

In a seventeenth aspect the present disclosure relates to an emulsion according to any of the aspects 1 to 15 for use in supplementing polyunsaturated fatty acids.

In an eighteenth aspect the present disclosure relates to a method for manufacturing the emulsion according to any of the aspects 1 to 15, comprising
a) providing an oil phase comprising one or more oils according to any of the aspects 5 to 7, vitamin A and optionally at least one pharmaceutically acceptable antioxidant and/or a pharmaceutically acceptable co-surfactant;
b) providing an aqueous phase comprising water for injection and optionally a pharmaceutically acceptable tonicity agent and/or an agent for pH adjustment and/or a pharmaceutically acceptable co-surfactant and/or a pharmaceutically acceptable co-solvent;
c) forming a pre-emulsion by mixing the oil phase provided in step a) with the aqueous phase provided in step b);
d) forming the emulsion by high-pressure homogenizing the pre-emulsion obtained in step c) and
e) sterilizing the emulsion obtained in step d),
wherein the at least one pharmaceutically acceptable emulsifier is added either in step a or in step b.

### DISCLOSURE

1) Emulsion for parenteral administration comprising 1000 to 65000, preferably 1500 to 60000, more preferably 20000 to 60000, most preferably 25000 to 55000 IU vitamin A per ml, being free of polysorbates and polyoxyethylen/polyoxypropylene block copolymers.
2) Emulsion according to embodiment 1 comprising 35000 to 55000, preferably 45000 to 55000 IU vitamin A per ml.
3) Emulsion according to embodiment 1 or 2, comprising vitamin A in a form other than beta-carotene, preferably in a form other than carotenoid.
4) Emulsion according to any of the preceding embodiments, comprising retinyl palmitate.
5) Emulsion according to any of the preceding embodiments, wherein the whole amount of vitamin A is provided in form of retinyl palmitate.
6) Emulsion according to any of the preceding embodiments, wherein vitamin A is the sole vitamin comprised as an active ingredient.
7) Emulsion according to any of the preceding embodiments, wherein the oil phase of the emulsion comprises one or more oils selected from the group consisting of soybean oil, olive oil, fish oil, fish oil extract, safflower oil, corn oil, sunflower oil, coconut oil, palm kernel oil, rapeseed oil and medium chain triglycerides (MCT).
8) Emulsion according to any of the preceding embodiments, wherein the oil phase of the emulsion comprises one or more oils selected from the group consisting of fish oil, fish oil extract, olive oil, soybean oil and MCT.
9) Emulsion according to any of the preceding embodiments, wherein the oil phase of the emulsion comprises fish oil, olive oil, soybean oil and MCT.
10) Emulsion according to any of the preceding embodiments, comprising a pharmaceutically acceptable tonicity agent, preferably glycerol.
11) Emulsion according to any of the preceding embodiments, comprising at least one co-solvent, preferably polyethylene glycol.
12) Emulsion according to any of the preceding embodiments comprising an agent for pH adjustment, preferably NaOH.
13) Emulsion according to any of the preceding embodiments comprising at least one pharmaceutically acceptable emulsifier, preferably lecithin.
14) Emulsion according to any of the preceding embodiments comprising a pharmaceutically acceptable co-surfactant, preferably an omega-9 fatty acid or a pharmaceutically acceptable salt thereof, more preferably oleic acid or sodium oleate.
15) Emulsion according to any of the preceding embodiments, comprising at least one pharmaceutically acceptable antioxidant, preferably selected from the group consisting of alpha-tocopherol, beta-tocopherol, gamma-tocopherol, ascorbic acid, ascorbic acid palmitate, an antioxidant obtained or obtainable from rosemary, rosemary extract and mixtures thereof.
16) Emulsion according to any of the preceding embodiments, wherein the emulsion is an oil-in-water emulsion and wherein the mean diameter of the oil droplets is between 100 and 350 nm.
17) Emulsion according to any of embodiments 1 to 16 for use in the treatment or prevention of a vitamin A deficiency.
18) Emulsion according to any of embodiments 1 to 16 for use in the treatment or prevention of a deficiency in polyunsaturated fatty acids.
19) Emulsion according to any of embodiments 1 to 16 for use in supplementing polyunsaturated fatty acids.
20) Method for the preparation of an emulsion according to any of embodiments 1 to 16 comprising
   a) providing an oil phase comprising one or more oils according to embodiment 7 to 9, vitamin A and optionally at least one pharmaceutically acceptable antioxidant and optionally a pharmaceutically acceptable co-surfactant,
   b) providing an aqueous phase comprising water for injection and optionally a pharmaceutically acceptable tonicity agent and/or an agent for pH-adjustment and/or a pharmaceutically acceptable co-solvent,
   c) forming a pre-emulsion by mixing the oil phase obtained in step a) with the aqueous phase obtained in step b),
   d) forming an emulsion by high pressure homogenizing the pre-emulsion obtained in step c,
   e) sterilizing the emulsion obtained in step d),
   wherein the at least one pharmaceutically acceptable emulsifier is added either in step a or in step b.

### Examples - mono vitamin preparations according to the present disclosure

### Examples 1 and 2

The emulsions were prepared from the ingredients listed in table 1.

The oil phases were prepared by mixing soybean oil, medium chain triglycerides, olive oil, fish oil, retinyl palmitate and alpha-tocopherol. The mixture was heated to 65 °C.
The aqueous phase was prepared by mixing water, glycerol and sodium oleate. The mixture was heated to 70 °C, and then egg lecithin was added.

The pre-emulsion was formed by adding the oil phase to the aqueous phase under continous agitation using a high shear mixer (Ultra Turrax T50) at a temperature of 68 °C.
The emulsion was formed by passing the pre-emulsion six times through a Niro Soavi TwinPanda 600 homogenizer at 500 bar at a temperature of 50 °C.
The pH was adjusted to 8.0 to 9.0.

Finally the emulsion was autoclaved at 121 °C for 15 minutes.

**Table 1:**

| **Ingredient** | **Amount [g]** | |
|---|---|---|
| | **Example 1** | **Example 2** |
| Soybean oil (oil phase) | 3.0 | |
| Medium chain triglycerides (oil phase) | 3.0 | |
| Olive oil (oil phase) | 2.5 | |
| Fish oil (oil phase) | 1.5 | |
| Egg lecithin (emulsifier) | 1.2 | |
| Retinyl palmitate (active ingredient) | 0.082 | 0.165 |
| Alpha-tocopherol (antioxidant) | 0.02 | |
| Sodium oleate (co-surfactant) | 0.03 | |
| Glycerol (tonicity agent) | 2.25 | |
| NaOH (1 M) (pH adjustment) | to adjust pH to 8.0 - 9.0 | |
| Water for injection (aqueous phase) | ad 100 | |

The oil droplets of the emulsion according to example 1 had a mean diameter of 172 nm when measured directly upon sterilization. The oil doplets of the emulsion according to example 2 had a mean diameter of 207 nm when measured directly upon sterilization using an LS 13 320 Laser Diffraction Particle Size Analyser (Beckman Coulter) according to USP <729>.

Stability data for both emulsions are shown in table 2.

As can be seen in table 2, all PFAT₅ values are below 0.05. Thus, both emulsions are stable for at least 2 weeks upon storage at 25 and 40 °C.

**Table 2:**

| **Storage time [w]** | **Storage T [°C]** | **pH** | | **Mean droplet diameter [nm]** | | **PFAT₅** | |
|---|---|---|---|---|---|---|---|
| | | 1 | **2** | **1** | **2** | **1** | **2** |
| 0 | - | 8.62 | 8.51 | 172 | 207 | 0.017 | 0.036 |
| 1 | 25 | 8.59 | 8.53 | 170 | 214 | 0.011 | 0.018 |
| | 40 | 8.48 | 8.29 | 169 | 213 | 0.013 | 0.023 |
| 2 | 25 | 8.60 | 8.49 | 179 | 232 | 0.005 | 0.014 |
| | 40 | 8.28 | 8.09 | 178 | 308 | 0.004 | 0.024 |

### Example 3

The emulsion was prepared from the ingredients listed in table 3.

The oil phase was prepared by mixing soybean oil, medium chain triglycerides, olive oil, fish oil, retinyl palmitate and alpha-tocopherol. The mixture was heated to 80 °C.

The aqueous phase was prepared by mixing water, glycerol and sodium oleate. The mixture was heated to 75 °C, and then egg lecithin was added.

The pre-emulsion was formed by adding the oil phase to the aqueous phase under continous agitation using a high shear mixer (Ultra Turrax T50) at a temperature of 75 °C.

The emulsion was formed by passing the pre-emulsion six times through a Niro Soavi TwinPanda 600 homogenizer at 500 bar at a temperature of 50 °C.
The pH was adjusted to 8.0 to 9.0.

Finally the emulsion was autoclaved at 121 °C for 15 minutes.

**Table 3:**

| **Ingredient** | **Amount [g]** |
|---|---|
| Soybean oil (oil phase) | 3.0 |
| Medium chain triglycerides (oil phase) | 3.0 |
| Olive oil (oil phase) | 2.5 |
| Fish oil (oil phase) | 1.5 |
| Retinyl palmitate (active ingredient) | 3.0 |
| Egg lecithin (emulsifier) | 1.2 |
| Sodium oleate (co-surfactant) | 0.03 |
| Glycerol (tonicity agent) | 2.25 |
| Alpha-tocopherol (antioxidant) | 0.02 |
| NaOH (1 M) (pH adjustment) | to adjust pH to 7.5 - 9.0 |
| Water for injection (aqueous phase) | ad 100 |

The oil droplets of the emulsion according to example 3 had a mean diameter of 280 nm when measured directly upon sterilization using an LS 13 320 Laser Diffraction Particle Size Analyser (Beckman Coulter) according to USP <729>.

Stability data are shown in table 4.

As can be seen in table 4, all PFAT₅ values are below 0.05. Thus, the emulsion is stable for at least 12 weeks upon storage at 25 and 40 °C respectively.

**Table 4:**

| **Storage time [w]** | **Storage T [°C]** | **pH** | **Mean droplet diameter [nm]** | **PFAT₅** |
|---|---|---|---|---|
| 0 | - | 8.37 | 280 | 0.030 |
| 1 | 25 | 8.30 | 340 | 0.017 |
| 2 | 25 | 8.31 | 340 | 0.020 |
| 12 | 40 | 7.53 | 260 | 0.034 |

### Example 4

The emulsion was prepared from the ingredients listed in table 5.

**Table 5:**

| **Ingredient** | **Amount [g]** |
|---|---|
| Fish oil (oil phase) | 7 |
| Retinyl palmitate (active ingredient) | 3.0 |
| Egg lecithin (emulsifier) | 1.2 |
| Sodium oleate (co-surfactant) | 0.03 |
| Glycerol (tonicity agent) | 2.25 |
| NaOH (1 M) (pH adjustment) | to adjust pH to 7.5 - 9.0 |
| Water for injection (aqueous phase) | ad 100 |

The oil phase was prepared by mixing fish oil and retinyl palmitate. The mixture was heated to 60 °C.

The aqueous phase was prepared by mixing water, glycerol and sodium oleate. The mixture was heated to 60 °C, and then egg lecithin was added.

The pre-emulsion was formed by adding the oil phase to the aqueous phase under continous agitation using a high shear mixer (Ultra Turrax T50) at a temperature of 60 °C.
The emulsion was formed by passing the pre-emulsion six times through a Niro Soavi TwinPanda 600 homogenizer at 500 bar at a temperature of 50 °C.

The pH was adjusted to 8.0 to 9.0.

Finally the emulsion was autoclaved at 121 °C for 15 minutes.

The oil droplets of the emulsion according to example 4 had a mean diameter of 216 nm when measured directly upon sterilization using an LS 13 320 Laser Diffraction Particle Size Analyser (Beckman Coulter) according to USP <729>.
Stability data are shown in table 6.

**Table 6:**

| **Storage time [w]** | **Storage T [°C]** | **pH** | **Mean droplet diameter [nm]** | **PFAT₅** |
|---|---|---|---|---|
| 0 | - | 7.97 | 216 | 0.019 |
| 2 | 25 | 7.81 | 233 | 0.009 |
| | 40 | 7.57 | 230 | 0.010 |
| 4 | 25 | 7.75 | 229 | 0.009 |
| | 40 | 7.48 | 228 | 0.012 |

As can be seen in table 6, all PFAT₅ values are below 0.05. Thus, the emulsion is stable for at least 4 weeks upon storage at 25 and 40 °C.

### Example 5

The emulsion was prepared from the ingredients listed in table 7.

The oil phase was prepared by mixing fish oil extract, oleic acid, mixed tocopherols and retinyl palmitate. The mixture was heated to 55 °C.

The aqueous phase was prepared by mixing water, glycerol and PEG. The mixture was heated to 70 °C, and then egg lecithin was added.

The pre-emulsion was formed by adding the oil phase to the aqueous phase under continous agitation using a high shear mixer (Ultra Turrax T50) at a temperature of 65 °C.
The emulsion was formed by passing the pre-emulsion six times through a Niro Soavi TwinPanda 600 homogenizer at 500 bar at a temperature of 60 °C.
The pH was adjusted to 8.0 to 9.0.

Finally the emulsion was autoclaved at 121 °C for 15 minutes.

**Table 7:**

| **Ingredient** | **Amount [g]** |
|---|---|
| Fish oil extract comprising 670 mg/g EPA and 115 mg/g DHA as triglycerides (obtained from Solutex S.L.) (oil phase) | 7 |
| Retinyl palmitate (active ingredient) | 3.0 |
| Egg lecithin (emulsifier) | 1.2 |
| Oleic acid (co-surfactant) | 0.15 |
| Glycerol (tonicity agent) | 2.25 |
| Polyethylene glycol (PEG) (co-solvent) | 1.0 |
| Mixed tocopherols (antioxidant) | 0.02 |
| NaOH (1 M) (pH adjustment) | to adjust pH to 7.5 - 9.0 |
| Water for injection (aqueous phase) | ad 100 |

The oil droplets of the emulsion according to example 5 had a mean diameter of 136 nm when measured directly upon sterilization using an LS 13 320 Laser Diffraction Particle Size Analyser (Beckman Coulter) according to USP <729>.

Stability data are shown in table 8.

**Table 8:**

| **Storage time [w]** | **Storage T [°C]** | **pH** | **Mean droplet diameter [nm]** | **PFAT₅** |
|---|---|---|---|---|
| 0 | - | 8.87 | 134 | 0.012 |
| 1 | 25 | 8.83 | 135 | 0.014 |
| 2 | 25 | 8.82 | 135 | 0.010 |
| 4 | 5 | 8.80 | 134 | 0.003 |
| | 25 | 8.81 | 133 | 0.012 |
| | 40 | 8.59 | 133 | 0.011 |

As can be seen in table 8, all PFAT₅ values are below 0.05. Thus, the emulsion is stable for at least 4 weeks upon storage at 5, 25 and 40 °C respectively.

### Example 6

The emulsion was prepared from the ingredients listed in table 9.

**Table 9:**

| **Ingredient** | **Amount [g]** |
|---|---|
| Fish oil (oil phase) | 7 |
| Retinyl palmitate (active ingredient) | 3.0 |
| Krill oil (emulsifier) | 1.8 |
| Sodium oleate (co-surfactant) | 0.03 |
| Glycerol (tonicity agent) | 2.5 |
| NaOH (1 M) (pH adjustment) | to adjust pH to 7.5 - 9.0 |
| Water for injection (aqueous phase) | ad 100 |

The oil phase was prepared by mixing fish oil, krill oil and retinyl palmitate. The mixture was heated to 60 °C.

The aqueous phase was prepared by mixing water, glycerol and sodium oleate. The mixture was heated to 60 °C.

The pre-emulsion was formed by adding the oil phase to the aqueous phase under continous agitation using a high shear mixer (Ultra Turrax T50) at a temperature of 60 °C.

The emulsion was formed by passing the pre-emulsion six times through a Niro Soavi TwinPanda 600 homogenizer at 500 bar at a temperature of 50 °C.

The pH was adjusted to 7.5 to 9.0.

Finally, the emulsion was autoclaved at 121 °C for 15 minutes.

The oil droplets of the emulsion according to example 6 had a mean diameter of 157 nm when measured directly upon sterilization using an LS 13 320 Laser Diffraction Particle Size Analyser (Beckman Coulter) according to USP <729>. The PFAT₅ value was 0.034.

## Claims

1. Emulsion for parenteral administration comprising 1000 to 65000, preferably 1500 to 60000, more preferably 20000 to 60000, most preferably 25000 to 55000 IU vitamin A per ml, being free of polysorbates and polyoxyethylen/polyoxypropylene block copolymers, wherein vitamin A is the sole vitamin comprised as an active ingredient, wherein the emulsion comprises at least one pharmaceutically acceptable emulsifier, and wherein this at least one pharmaceutically acceptable emulsifier is lecithin or krill oil.

2. Emulsion according to claim 1 comprising retinyl palmitate.

3. Emulsion according to any of the preceding claims, wherein the whole amount of vitamin A is provided in form of retinyl palmitate.

4. Emulsion according to any of the preceding claims, wherein the oil phase of the emulsion comprises one or more oils selected from the group consisting of soybean oil, olive oil, fish oil, fish oil extract, safflower oil, corn oil, sunflower oil, coconut oil, palm kernel oil, rapeseed oil and medium chain triglycerides (MCT).

5. Emulsion according to any of the preceding claims, wherein the oil phase of the emulsion comprises fish oil, olive oil, soybean oil and MCT.

6. Emulsion according to any of the preceding claims comprising a pharmaceutically acceptable tonicity agent, preferably glycerol.

7. Emulsion according to any of the preceding claims comprising at least one co-solvent, preferably polyethylene glycol.

8. Emulsion according to any of the preceding claims further comprising an agent for pH adjustment, preferably NaOH.

9. Emulsion according to any of the preceding claims comprising a pharmaceutically acceptable co-surfactant, preferably an omega-9 fatty acid or a pharmaceutically acceptable salt thereof, more preferably oleic acid or sodium oleate.

10. Emulsion according to any of the preceding claims, wherein the emulsion comprises at least one pharmaceutically acceptable antioxidant, preferably selected from the group consisting of alpha-tocopherol, beta-tocopherol, gamma-tocopherol, ascorbic acid, ascorbic acid palmitate, an antioxidant obtained or obtainable from rosemary, rosemary extract and mixtures thereof.

11. Emulsion according to any of the preceding claims, wherein the emulsion is an oil-in-water emulsion and wherein the mean diameter of the oil droplets is between 100 and 350 nm.

12. Emulsion according to any of claims 1 to 11 for use in the treatment or prevention of a vitamin A deficiency.

13. Method for the preparation of an emulsion according to any of claims 1 to 11 comprising
a) providing an oil phase comprising one or more oils selected from the group according to claim 6 or 7, vitamin A and optionally a pharmaceutically acceptable co-surfactant,
b) providing an aqueous phase comprising water for injection and optionally a pharmaceutically acceptable tonicity agent and/or an agent for pH-adjustment and/or a pharmaceutically acceptable co-solvent,
c) forming a pre-emulsion by mixing the oil phase obtained in step a) with the aqueous phase obtained in step b),
d) forming an emulsion by high pressure homogenizing the pre-emulsion obtained in step c,
e) sterilizing the emulsion obtained in step d),
wherein the at least one pharmaceutically acceptable emulsifier is added either in step a or in step b.

## Patentansprüche

1. Emulsion zur parenteralen Verabreichung umfassend 1000 bis 65000, vorzugsweise 1500 bis 60000, weiter vorzugsweise 20000 bis 60000, am meisten bevorzugt 25000 bis 55000 IE Vitamin A pro ml, welche frei von Polysorbaten und Polyoxyethylen/Polyoxypropylen-Blockcopolymeren ist, wobei Vitamin A das einzige Vitamin ist, welches als Wirkstoff umfasst ist, wobei die Emulsion mindestens einen pharmazeutisch akzeptablen Emulgator umfasst, und wobei dieser mindestens eine pharmazeutisch akzeptable Emulgator Lecithin oder Krillöl ist.

2. Emulsion nach Anspruch 1, umfassend Retinylpalmitat.

3. Emulsion nach einem der vorhergehenden Ansprüche, wobei die Gesamtmenge von Vitamin A in Form von Retinylpalmitat bereitgestellt wird.

4. Emulsion nach einem der vorhergehenden Ansprüche, wobei die Ölphase der Emulsion ein oder mehrere Öle ausgewählt aus der Gruppe bestehend aus Sojabohnenöl, Olivenöl, Fischöl, Fischölextrakt, Distelöl, Maisöl, Sonnenblumenöl, Kokosöl, Palmkernöl, Rapsöl und mittelkettigen Triglyceriden (MCT) umfasst.

5. Emulsion nach einem der vorhergehenden Ansprüche, wobei die Ölphase der Emulsion Fischöl, Olivenöl, Sojabohnenöl und MCT umfasst.

6. Emulsion nach einem der vorhergehenden Ansprüche, umfassend ein pharmazeutisch akzeptables Tonizitätsmittel, vorzugsweise Glycerin.

7. Emulsion nach einem der vorhergehenden Ansprüche, umfassend mindestens ein Co-Lösungsmittel, vorzugsweise Polyethylenglycol.

8. Emulsion nach einem der vorhergehenden Ansprüche, zusätzlich umfassend ein Mittel für die Einstellung des pH-Wertes, vorzugsweise NaOH.

9. Emulsion nach einem der vorhergehenden Ansprüche, umfassend ein pharmazeutisch akzeptables Co-Tensid, vorzugsweise eine Omega-9-Fettsäure oder ein pharmazeutisch akzeptables Salz derselben, weiter vorzugsweise Ölsäure oder Natriumoleat.

10. Emulsion nach einem der vorhergehenden Ansprüche, wobei die Emulsion mindestens ein pharmazeutisch akzeptables Antioxidans, vorzugsweise ausgewählt aus der Gruppe bestehend aus Alpha-Tocopherol, Beta-Tocopherol, Gamma-Tocopherol, Ascorbinsäure, Ascorbinsäurepalmitat, ein Antioxidans, welches aus Rosmarin, Rosmarinextrakt und Mischungen derselben erhalten wird oder erhältlich ist, umfasst.

11. Emulsion nach einem der vorhergehenden Ansprüche, wobei die Emulsion eine Öl-in-Wasser-Emulsion ist und wobei der mittlere Durchmesser der Öltröpfchen zwischen 100 und 350 nm liegt.

12. Emulsion nach einem der Ansprüche 1 bis 11 für die Verwendung bei der Behandlung oder Vorbeugung eines Vitamin A-Mangels.

13. Verfahren zur Zubereitung einer Emulsion nach einem der Ansprüche 1 bis 11, umfassend
a) das Bereitstellen einer Ölphase umfassend ein oder mehrere Öle ausgewählt aus der Gruppe nach Anspruch 6 oder 7, Vitamin A und wahlweise ein pharmazeutisch akzeptables Co-Tensid,
b) das Bereitstellen einer wässrigen Phase umfassend Wasser für Injektionszwecke und wahlweise ein pharmazeutisch akzeptables Tonizitätsmittel und/oder ein Mittel für die Einstellung des pH-Wertes und/oder ein pharmazeutisch akzeptables Co-Lösungsmittel,
c) das Bilden einer Voremulsion mittels des Mischens der in Schritt a) erhaltenen Ölphase mit der in Schritt b) erhaltenen wässrigen Phase,
d) das Bilden einer Emulsion mittels der Hochdruckhomogenisierung der in Schritt c erhaltenen Voremulsion,
e) das Sterilisieren der in Schritt d) erhaltenen Emulsion,
wobei der mindestens eine pharmazeutisch akzeptable Emulgator entweder in Schritt a oder in Schritt b hinzugefügt wird.

## Revendications

1. Emulsion pour administration parentérale comprenant de 1000 à 65000, de préférence de 1500 à 60000, plus de préférence de 20000 à 60000, le plus préférablement de 25000 à 55000 UI de vitamine A par ml, étant libre de polysorbates et de copolymères séquencés de polyoxyéthylène/polyoxypropylène, dans laquelle la vitamine A est la seule vitamine comprise comme principe actif, dans laquelle l'émulsion comprend au moins un émulsifiant pharmaceutiquement acceptable, et dans laquelle ledit au moins un émulsifiant pharmaceutiquement acceptable est la lécithine ou l'huile de krill.

2. Emulsion selon la revendication 1, comprenant du palmitate de rétinyle.

3. Emulsion selon l'une quelconque des revendications précédentes, dans laquelle la quantité intégrale de vitamine A est fournie sous forme de palmitate de rétinyle.

4. Emulsion selon l'une quelconque des revendications précédentes, dans laquelle la phase huileuse de l'émulsion comprend une ou plusieurs huiles choisies parmi le groupe composé d'huile de soja, d'huile d'olive, d'huile de poisson, d'extrait d'huile de poisson, d'huile de carthame, d'huile de maïs, d'huile de tournesol, d'huile de coco, d'huile de palmiste, d'huile de colza et de triglycérides à chaîne moyenne (TCM).

5. Emulsion selon l'une quelconque des revendications précédentes, dans laquelle la phase huileuse de l'émulsion comprend de l'huile de poisson, de l'huile d'olive, de l'huile de soja et des TCM.

6. Emulsion selon l'une quelconque des revendications précédentes, comprenant un agent de tonicité pharmaceutiquement acceptable, de préférence du glycérol.

7. Emulsion selon l'une quelconque des revendications précédentes, comprenant au moins un co-solvant, de préférence du polyéthylène glycol.

8. Emulsion selon l'une quelconque des revendications précédentes, comprenant en outre un agent pour ajustement du pH, de préférence du NaOH.

9. Emulsion selon l'une quelconque des revendications précédentes, comprenant un co-tensioactif pharmaceutiquement acceptable, de préférence un acide gras oméga-9 ou un sel pharmaceutiquement acceptable de celui-ci, plus de préférence de l'acide oléique ou de l'oléate de sodium.

10. Emulsion selon l'une quelconque des revendications précédentes, dans laquelle l'émulsion comprend au moins un antioxydant pharmaceutiquement acceptable, de préférence choisi parmi le groupe composé d'alpha-tocophérol, bêta-tocophérol, gamma-tocophérol, acide ascorbique, palmitate d'acide ascorbique, un antioxydant obtenu ou obtenable à partir de romarin, extrait de romarin et des mélanges de ceux-ci.

11. Emulsion selon l'une quelconque des revendications précédentes, dans laquelle l'émulsion est une émulsion d'huile-dans-l'eau et dans laquelle le diamètre moyen des goutes d'huile se trouve entre 150 et 350 nm.

12. Emulsion selon l'une quelconque des revendications 1 à 11, pour son utilisation dans le traitement ou la prévention d'une carence en vitamine A.

13. Procédé pour la préparation d'une émulsion selon l'une quelconque des revendications 1 à 11, comprenant
a) fournir une phase huileuse comprenant une ou plusieurs huiles choisies parmi le groupe selon la revendication 6 ou 7, de la vitamine A et optionnellement un co-tensioactif pharmaceutiquement acceptable,
b) fournir une phase aqueuse comprenant de l'eau pour injection, et optionnellement un agent de tonicité pharmaceutiquement acceptable et/ou un agent pour ajustement du pH et/ou un co-solvant pharmaceutiquement acceptable,
c) former une pré-émulsion en mélangeant la phase huileuse obtenue dans l'étape a) avec la phase aqueuse obtenue dans l'étape b),
d) former une émulsion par homogénéisation à haute pression de la pré-émulsion obtenue dans l'étape c),
e) stériliser l'émulsion obtenue dans l'étape d),
dans lequel l'au moins un émulsifiant pharmaceutiquement acceptable est ajouté soit à l'étape a soit à l'étape b.
